# EUROPEAN PATENT SPECIFICATION

(11) **EP 1 594 970 B1**
(45) Date of publication and mention of the grant of the patent: **30.07.2008**
(21) Application number: 04712013.4
(22) Date of filing: 18.02.2004
(51) Int. Cl.: C12N 15/86

(54) **RECOMBINANT MVA AND METHOD FOR GENERATION THEREOF**
REKOMBINANTES MVA UND VERFAHREN ZUR ERZEUGUNG DAVON
VIRUS ANKARA MODIFIE (MVA) RECOMBINANT ET PROCEDE DE PRODUCTION

(30) Priority: 18.02.2003 US 448231 P
(43) Date of publication of application: 16.11.2005
(73) Proprietor: Helmholtz Zentrum München Deutsches Forschungszentrum für Gesundheit und Umwelt (GmbH), 85764 Neuherberg (DE)
(72) Inventor: STAIB, Caroline, 80804 Muenchen (DE); LOEWEL, Marianne, 80807 Muenchen (DE); ERFLE, Volker, 81679 Muenchen (DE); SUTTER, Gerd, 80803 Muenchen (DE)
(74) Representative: Behnisch, Werner
(86) International application number: PCT/EP2004/001554
(87) International publication number: WO 2004/074493

(56) References cited:
- WO-A-02/090550
- STAIB ET AL.: "TRANSIENT HOST RANGE SELECTION FOR GENETIC ENGINEERING OF MODIFIED VACCINIA VIRUS ANKARA" BIOTECHNIQUES, EATON PUBLISHING, NATICK, US, vol. 28, no. 6, June 2000 (2000-06), pages 1137-1148, XP001026101 ISSN: 0736-6205
- MEYER H ET AL: "MAPPING OF DELETIONS IN THE GENOME OF THE HIGHLY ATTENUATED VACCINIA VIRUS MVA AND THEIR INFUENCE ON VIRULENCE" JOURNAL OF GENERAL VIROLOGY, READING, BERKS, GB, vol. 72, 1991, pages 1031-1038, XP000952390 ISSN: 0022-1317

## Description

The present invention relates to MVA mutants, which can be used for the generation of recombinant MVA viruses, as well as host cells, which have been infected with these mutant MVA viruses. The present invention further relates to DNA-vector constructs, and a method for the generation of recombinant MVA by using the mutant MVA viruses and the DNA-vector constructs.

Vaccinia virus (VV) belongs to the genus Orthopoxvirus of the family of poxviruses. Certain strains of vaccinia virus have been used for many years as live vaccine to immunize against smallpox, for example the Elstree strain of the Lister Institute in the UK. Because of the complications which may derive from the vaccination (Schär, Zeitschr. für Präventivmedizin 18, 41-44 [1973]), and since the declaration in 1980 by the WHO that smallpox had been eradicated nowadays only people at high risk are vaccinated against smallpox.

Vaccinia viruses have also been used as vectors for production and delivery of foreign antigens (Smith et al., Biotechnology and Genetic Engineering Reviews 2, 383-407 [1984]). This entails DNA sequences (genes) which code for foreign antigens being introduced, with the aid of DNA recombination techniques, into the genome of the vaccinia viruses. If the gene is integrated at a site in the viral DNA which is non -essential for the life cycle of the virus, it is possible for the newly produced recombinant vaccinia virus to be infectious, that is to say able to infect foreign cells and thus to express the integrated DNA sequence (EP Patent Applications No. 83,286 and No. 110,385). The recombinant vaccinia viruses prepared in this way can be used, on the one hand, as live vaccines for the prophylaxis of infections, on the other hand, for the preparation of heterologous proteins in eukaryotic cells.

Vaccinia virus is amongst the most extensively evaluated live vectors and has particular features in support of its use as recombinant vaccine: It is highly stable, cheap to manufacture, easy to administer, and it can accommodate large amounts of foreign DNA. It has the advantage of inducing both antibody and cytotoxic responses, and allows presentation of antigens to the immune system in a more natural way, and it was successfully used as vector vaccine protecting against infectious diseases in a broad variety of animal models. Additionally, vaccinia vectors are extremely valuable research tools to analyze structure-function relationships of recombinant proteins, determine targets of humoral and cell-mediated immune responses, and investigate the type of immune defense needed to protect against a specific disease.

However, vaccinia virus is infectious for humans and its use as expression vector in the laboratory has been affected by safety concerns and regulations. Furthermore, possible future applications of recombinant vaccinia virus e.g. to generate recombinant proteins or recombinant viral particles for novel therapeutic or prophylactic approaches in humans, are hindered by the productive replication of the recombinant vaccinia vector. Most of the recombinant vaccinia viruses described in the literature are based on the Western Reserve (WR) strain of vaccinia virus. On the other hand, it is known that this strain is highly neurovirulent and is thus poorly suited for use in humans and animals (Morita et al., Vaccine 5, 65-70 [1987]).

Concerns with the safety of standard strains of VV have been addressed by the development of vaccinia vectors from highly attenuated virus strains which are characterized by their restricted replicative capacity *in vitro* and their avirulence *in vivo.* Strains of viruses specially cultured to avoid undesired side effects have been known for a long time. Thus, it has been possible, by long-term serial passages of the Ankara strain of vaccinia virus (CVA) on chicken embryo fibroblasts, to culture a modified vaccinia virus Ankara (MVA) (for review see Mayr, A., Hochstein-Mintzel, V. and Stickl, H. (1975) Infection 3, 6-14; Swiss Patent No. 568 392). The MVA virus was deposited in compliance with the requirements of the Budapest Treaty at CNCM (Institut Pasteur, Collectione Nationale de Cultures de Microorganisms, 25, rue de Docteur Roux, 75724 Paris Cedex 15) on Dec. 15, 1987 under Depositary No. I-721.

The MVA virus has been analysed to determine alterations in the genome relative to the wild type CVA strain. Six major deletions (deletion I, II, III, IV, V, and VI ) have been identified (Meyer, H., Sutter, G. and Mayr A. (1991) J. Gen. Virol. 72, 1031-1038). This modified vaccinia virus Ankara has only low virulence, that is to say it is followed by no side effects when used for vaccination. Hence it is particularly suitable for the initial vaccination of immunocompromised subjects. The excellent properties of the MVA strain have been demonstrated in a number of clinical trials (Mayr et al., Zbl. Bakt. Hyg. I, Abt. Org. B 167, 375-390 [1987], Stickl et al., Dtsch. med. Wschr. 99, 2386-2392 [1974]).

Modified vaccinia virus Ankara (MVA) is a valuable tool as safe viral vector for expression of recombinant genes and can be used for such different purposes as the *in vitro* study of protein functions or the *in vivo* induction of antigen-specific cellular or humoral immune responses. A major advantage of MVA is to allow for high level gene expression despite being replication defective in human and most mammalian cells. MVA as a vaccine has an excellent safety track-record, can be handled under biosafety level 1 conditions and has proven to be immunogenic and protective when delivering heterologous antigens in animals (1-8), and first human candidate vaccines have proceeded into clinical trials (9-11).

While there is increasing demand for the evaluation of new constructs, the generation of MVA vectors is different when compared to replication competent recombinant vaccinia virus, due to growth deficiency of the virus and diminished cytopathic effects produced. Quick and easy methods for generating recombinant MVA are necessary to allow for comparative evaluation of multiple candidate constructs.

Compared to its parental strain, MVA has deletions that consist of about 15 percent (30,000 base pairs) of its former genome, including most of the *KIL* gene. Only a fragment of a length of 263 bp is still present in the MVA genome. The MVA *KIL* gene sequences represent the first 263 bp of the ORF 022L in the MVA genome at position nt 20685-20981 as described in Antoine, G., F. Scheiflinger, F. Dorner, and F. G. Falkner. 1998. The complete genomic sequence of the modified vaccinia Ankara strain and a comparison with other orthopoxviruses can be found in: Virology 244:365-396.

Previously, an easy and highly efficient method for generation of recombinant MVA based on selection for transient expression of the vaccinia virus host range gene *K1L* has been developed. This method is based on selection of recombinant MVA by transient host range gene expression (12), using the vaccinia virus *KIL* gene function as stringent marker to rescue MVA growth on rabbit kidney RK-13 cells. The construction and use of new MVA vector plasmids was described which carry an expression cassette of the vaccinia virus host range gene *K1L* as transient selectable marker. These plasmids allow either stable insertion of additional recombinant genes into the MVA genome or precisely targeted mutagenesis of MVA genomic sequences. Repetitive DNA sequences flanking the *K1L* gene were designed to remove the marker gene from the viral genome by homologous recombination under non-selective growth conditions.

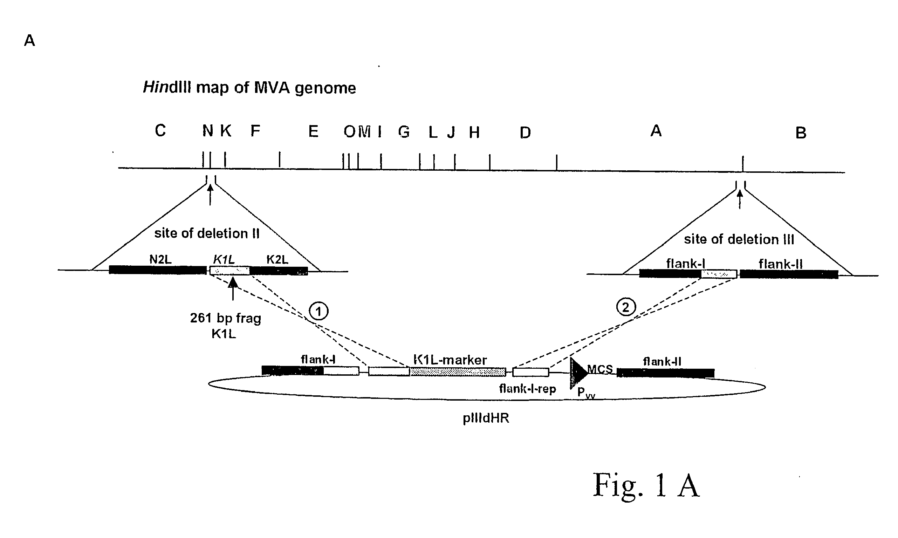

When using this straightforward selection protocol for construction of multiple recombinant MVA carrying heterologous gene sequences including the green fluorescent protein (gfp) gene from the jellyfish *Aequorea victoria,* the inventors observed that part of the isolated recombinant MVA was able to grow on RK13 cells but did not express the target gene of interest. Upon molecular analysis of multiple isolated MVA two different failures were detected: (i) at the insertion site for the recombinant gene presence of only the *KIL* marker gene, no recombinant gene inserted, (ii) the region of natural deletion II within the MVA genome is affected/truncated (Fig. 1A). The first observation is due to an initial single cross over event between flank I gene sequences in the MVA genome and flank I repeat sequences of the transfer plasmid of the prior art (12), followed by a second event of homologous recombination between remaining flank I sequences resulting in stable insertion of only the *K1L* marker sequence into the MVA genome. The second observation occurs, when homologous recombination events take place between *K1L* gene sequences in MVA and the transfer plasmid. The possibility of the latter recombination event has also been suggested by Tscharke & Smith (13).

Therefore, it is an object of the present invention to provide an improved method of producing recombinant MVA, which overcomes the above mentioned problems. It is a further object of the present invention to provide MVA mutants and DNA vector constructs, which can be used in such a recombination method.

This is accomplished by the subject-matter of the independent claims. Preferred embodiments of the present invention are set forth in the dependent claims.

According to the present invention, a new MVA mutant is provided, which is characterized in that the MVA *KIL* gene sequences (comprising only a fragment as defined below) and preferably its promoter sequences or a functional part thereof have been inactivated in the MVA genome by mutation or deletion or both.

As mentioned above, only a fragment of a length of 264 bp of the *K1L* gene is still present in the MVA genome (coding sequence). Furthermore, the respective regulatory sequences are present, which comprise about 100 additional bp. The MVA *K1L* gene sequences are part of ORF 022L in the MVA genome at position nt 20685-20981 as described in Antoine, G., F. Scheiflinger, F. Dorner, and F. G. Falkner. 1998. Thus, it is to be understood that inactivation of a functional part of the MVA *K1L* gene sequences and regulatory sequences will be sufficient in order to accomplish the object of the present invention. "Inactivation" means alterations in the sequences to avoid homologous recombination between *K1L* gene sequences in MVA and the transfer plasmid (DNA vector construct), causing an affection/truncation in the region of natural deletion II within the MVA genome (and resulting in a lack of expression of the gene of interest).

According to a preferred embodiment, the MVA *K1L* gene, preferably together with its promoter sequence, or a functional part thereof has been inactivated by deletion from the viral genome. Alternatively, a recombinant MVA defective in *K1L* sequence function may be generated by sequence mutagenesis, e.g. insertional mutagenesis, leading to the inactivation of *K1L* sequences through inhibition of *K1L* gene-specific recombination. This recombinant MVA can advantageously be used in a method for the introduction of foreign genes and subsequent selection of transfected strains, i.e. in a method for the generation of recombinant MVA.

As mentioned above, the inactivation in the MVA genome means generally a degree of inactivation, which results in avoiding a homologous recombination between *K1L* gene sequences in MVA and the transfer plasmid (vector construct). In case of mutation, the degree of homology between the inactivated *K1L* sequence of the invention and the VV *K1L* gene should not exceed 50% and preferably is in the range between 10-50, more preferably 15-40 and most preferably 20-30% homology. It is also possible that the homology is 0%.

In case of inactivation by deletion, the deleted fragment should at least comprise 100, more preferably 150 or 200 bp of the MVA K1L gene. It is, as mentioned above, also possible to delete all 264 bp. Furthermore, also the regulatory sequences should be deleted to a greater extent in order to avoid homologous recombination events. It is possible to delete also the whole K1L sequence comprising both, the coding sequence as well as the regulatory sequences (i.e. a deletion of about 360 bp).

Using recently established methodology of transfection of plasmid DNA into primary chicken embryo fibroblasts (CEF) infected with MVA, followed by selection of β-galactosidase producing viruses in the presence of mycophenolic acid (Sutter, G. and B. Moss. 1992, PNAS USA 89:10847-10851.) mutant MVA were generated having *K1L* coding sequences deleted from the viral genome.

The principle underlying the invention is that as long as MVA is present on RK-13 cells without the appropriate DNA sequences having been introduced (*K1L* coding sequences and optionally further DNA sequences, which are, e.g. coding for heterologous proteins), a replication does not occur. Therefore, the present method/MVA mutant/DNA construct is suitable for the selection of recombinant MVA and therefore serves as a tool for the effective generation of recombinant MVA.

As used herein, the term "recombinant MVA" means those MVA, which have been genetically altered, e.g. by DNA recombination techniques and which are provided for the use, for example, as a vaccine or as an expression vector.

According to the present invention, the recombinant MVA vaccinia viruses can be prepared as follows. However, it is to be understood that a person skilled in the art can make alterations within the scope of the present invention and his state of the art knowledge. Furthermore, the literature cited herein is incorporated by reference.

A DNA-construct which contains a DNA-sequence which codes for the Vaccinia Virus (VV) *K1L* protein or a *K1L*-derived polypeptide and a DNA sequence encoding a foreign polypeptide both flanked by DNA sequences flanking a non-essential site, e. g. a naturally occuring deletion, e.g. deletion III, within the MVA genome, is introduced into cells, preferably eucaryotic cells. Preferably, avian, mammalian and human cells are used. Preferred eucaryotic cells are BHK-21 (ATCC CCL-10), BSC-1 (ATCC CCL-26), CV-1 (ECACC 87032605) or MA104 (ECACC 85102918) cells) productively infected with mutant MVA according the invention, to allow homologous recombination. Further preferred host cells are chicken fibroblast cells, quail fibroblast cells, QT-9 cells, Vero cells, MRC-5 cells, B-cells or human primary cells (e.g. primary fibroblast cells, dendritic cells).

As mentioned above, the DNA construct comprises a sequence coding for the VV K1L gene or for a functionally equivalent gene. Functionally equivalent in the meaning of this invention are such nucleic acids, which contain one or more substitutions, insertions and or deletions when compared to the nucleic acids of the VV K1L gene without altering its function. These lack preferably one, but also 2, 3, 4, or more nucleotides 5' or 3' or within the nucleic acid sequence, or these nucleotides are replaced by others. According to the invention such VV K1L equivalent protein coding nucleic acids can show for example at least about 80%, more typically at least about 90% or 95% sequence identity to the nucleic acids of the original VV K1L gene (see above).

In this context, in particular variants of the VV K1L gene coding for an equivalent VV K1L protein, for example deletions, insertions and/or substitutions in the sequence, which cause for so-called "silent" changes, are considered to be part of the invention.

For example, such changes in the nucleic acid sequence are considered to cause a substitution with an equivalent amino acid. Preferably are such amino acid substitutions the result of substitutions which substitute one amino acid with a similar amino acid with similar structural and/or chemical properties, i.e. conservative amino acid substitutions.

Amino acid substitutions can be performed on the basis of similarity in polarity, charges, solubility, hydrophobic, hydrophilic, and/or amphipathic (amphiphil) nature of the involved residues. Examples for hydrophobic amino acids are alanine, leucine, isoleucine, valine, proline, phenylalanine, tryptophan and methionine. Polar, neutral amino acids include glycine, serine, threonine, cysteine, thyrosine, asparagine and glutamine. Positively (basic) charged amino acids include arginine, lysine and histidine. And negatively charged amino acids include aspartic acid and glutamic acid.

"Insertions" or "deletions" usually range from one to five amino acids. The allowed degree of variation can be experimentally determined via methodically applied insertions, deletions or substitutions of amino acids in a polypeptide molecule using recombinant DNA methods. The resulting variants can be tested for their biological activity.

Nucleotide changes, which affect the N-terminal and C-terminal part of the protein, often do not change the protein activity, because these parts are often not involved in the biological activity. Each of the suggested modifications is in range of the current state of the art, and under the retention of the biological activity of the encoded products.

Once the DNA-construct has been introduced into the eukaryotic cell and the *K1L* coding DNA and foreign DNA has recombined with the viral DNA, it is possible to isolate the desired recombinant vaccinia virus MVA upon passage in cells that require *K1L* function to support virus growth, e.g. RK-13 cells. The cloning of the recombinant viruses is possible in a manner known as plaque purification (compare Nakano et al., Proc. Natl. Acad. Sci. USA 79, 1593-1596 [1982], Franke et al., Mol. Cell. Biol. 1918-1924 [1985], Chakrabarti et al., Mol. Cell. Biol. 3403-3409 [1985], Fathi et al., Virology 97-105 [1986]).

The DNA-construct to be inserted can be linear or circular. A circular DNA is preferably used. It is particularly preferable to use a plasmid.

The DNA-construct may contain sequences flanking the left and the right side of a non-essential site, e.g. the site of deletion III, within the MVA genome (Sutter, G. and Moss, B. (1992) Proc. Natl. Acad. Sci. USA 89, 10847-10851), the site of the engineered *K1L* deletion within the MVA genome or any non-essential site within the genome of mutant MVA according to this invention.

The foreign DNA sequence may be inserted between the sequences flanking the non-essential site, e.g. the naturally occuring deletion.

The foreign DNA sequence can be a gene coding for a therapeutic polypeptide, e.g secreted proteins, e.g. polypeptides of antibodies, chemokines, cytokines or interferons, or a polypeptide from a pathogenic agent which can be used preferably for vaccination purposes or for the production of therapeutic or scientific valuable polypeptides. Pathogenic agents are to be understood to be viruses, bacteria and parasites which may cause a disease, as well as tumor cells which multiply unrestrictedly in an organism and may thus lead to pathological growths. Examples of such pathogenic agents are described in Davis, B.D. et al., (Microbiology, 3rd ed., Harper International Edition). Preferred genes of pathogenic agents are those of influenza viruses, of measles and respiratory syncytial viruses, of dengue viruses, of human immunodeficiency viruses, for example HIV I and HIV II, of human hepatitis viruses, e.g. HCV and HBV, of herpes viruses, of papilloma viruses, of the malaria parasite Plasmodium falciparum, and of the tuberculosis-causing Mycobacteria.

Preferred genes encoding tumor associated antigens are those of melanoma-associated differentiation antigens, e.g. tyrosinase, tyrosinase-related proteins 1 and 2, of cancer testes antigens, e.g. MAGE-1,-2,-3, and BAGE, of non-mutated shared antigens overexpressed on tumors, e.g. Her-2/neu, MUC-1, and p53.

In order for it to be possible for the foreign DNA sequence or the gene to be expressed, it is necessary for regulatory sequences, which are required for the transcription of the gene, to be present on the DNA. Such regulatory sequences (called promoters) are known to those skilled in the art, for example a vaccinia virus specific promoter as that of the vaccinia 11 kDa gene as are described in EP-A-198,328, and those of the 7.5 kDa gene (EP-A-110,385) or a heterologous poxvirus promoter which allows for vaccinia virus specific transcription, or a synthetic promoter which allows for vaccinia virus specific transcription.

According to a preferred embodiment, the DNA-vector construct of the invention comprises the following, functionally linked components:
- the *K1L* coding sequence of VV under transcriptional control of its authentic promoter (*K1L* marker gene), of a vaccinia virus specific promoter, or of a heterologous poxvirus promoter
- two DNA sequences flanking the *K1L* marker gene for subsequent removal of the marker from recombinant MVA by homologous recombination, preferably two identical inert (e.g. E. coli *lacZ* derived) DNA fragments,
- a multiple cloning site, in which a heterologous gene has been optionally ligated,
- a vaccinia virus specific promoter, or a heterologous poxvirus promoter which allows for vaccinia virus specific transcription, or a synthetic promoter which allows for vaccinia virus specific transcription,
which components are flanked by two MVA-DNA sequences, which are essential to target insertion of foreign genes to any non-essential site within the genome of mutant MVA according to the invention as described above, to the site of deletion III within the MVA genome, or to the site of the engineered K1L deletion within the MVA genome.

The DNA-construct can be introduced into the cells by transfection, for example by means of calcium phosphate precipitation (Graham et al., Virol. 52, 456-467 [1973]; Wigler et al., Cell 777-785 [1979]), by means of electroporation (Neumann et al., EMBO J. 1, 841-845 [1982]), by microinjection (Graessmann et al., Meth. Enzymology 101, 482-492 (1983), by means of liposomes (Straubinger et al., Methods in Enzymology 101, 512-527 (1983), by means of spheroplasts (Schaffner, Proc. Natl. Acad. Sci. USA 77, 2163-2167 (1980) or by other methods known to those skilled in the art. Transfection by means of calcium phosphate precipitation is preferably used.

To prepare vaccines, the MVA vaccinia viruses generated according to the invention are converted into a physiologically acceptable form. This can be done based on the many years of experience in the preparation of vaccines used for vaccination against smallpox (Kaplan, Br. Med. Bull. 25, 131-135 [1969]). Typically, about 10⁶-10⁸ particles of the recombinant MVA are freeze-dried in 100ml of phosphate-buffered saline (PBS) in the presence of 2% peptone and 1% human albumin in an ampoule, preferably a glass ampoule. The lyophilisate can contain extenders (such as mannitol, dextran, sugar, glycine, lactose or polyvinylpyrrolidone) or other aids (such as antioxidants, stabilizers, etc.) suitable for parenteral administration. The glass ampoule is then sealed and can be stored, preferably at temperatures below -20°C, for several months.

For vaccination the lyophilisate can be dissolved in 0.1 to 0.2 ml of aqueous solution, preferably physiological saline, and administered parenterally, for example by intradermal inoculation. The vaccine according to the invention is preferably injected intracutaneously. Slight swelling and redness, sometimes also itching, may be found at the injection site (Stickl et al., supra). The mode of administration, the dose and the number of administrations can be optimized by those skilled in the art in a known manner. It is expedient where appropriate to administer the vaccine several times over a lengthy period in order to obtain a high level immune responses against the foreign antigen.

As a summary, the method of the present invention for the generation of recombinant MVA comprises the following steps: Infecting the host cells as described above with the mutant MVA, transfecting the host cells with a DNA-vector construct of the present invention and selecting restored MVA by growth on rabbit kidney RK-13 cells, or any other cell type that essentially requires *K1L* gene function to allow for productive growth of MVA or mutant MVA as in claim 1.

### BRIEF DESCRIPTION OF THE DRAWINGS:

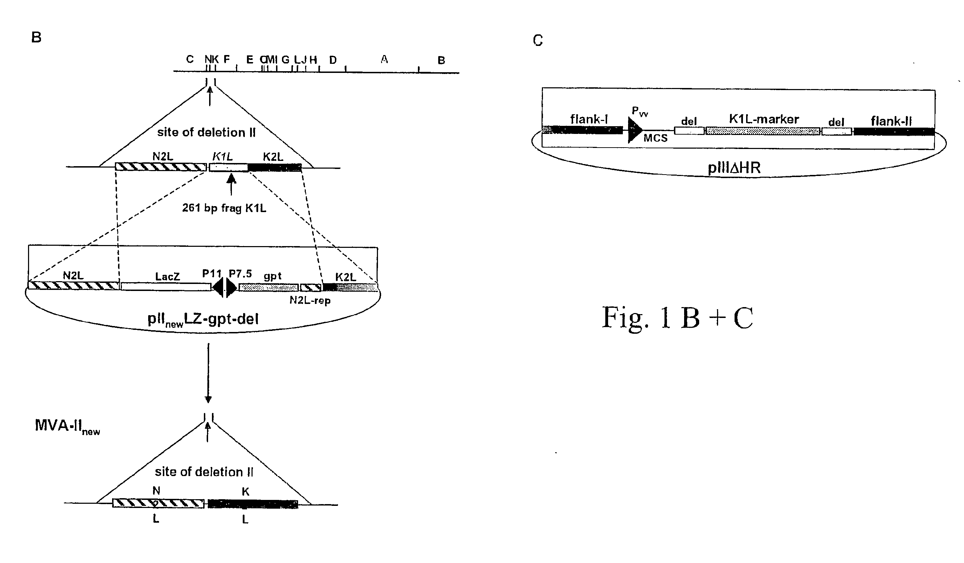

**Figure 1****: Schematics of possible pit-falls of *K1L*-based host range selection and their elimination.** (A) Schematic maps of the MVA genome (*Hind*III restriction map) and the vector plasmid pIIIdHR. Site of deletion II: Representation of ORFs, including fragmented *K1L* gene (hatched boxes). Site of deletion III: Flank-I and flank-II refer to MVA-DNA sequences which are essential to target insertion of foreign genes to the site of deletion III within the MVA genome. Flank-I-rep indicates the position of a 283 bp repetitive MVA-DNA fragment homologous to the right end of flank-I which allows deletion of the *K1L* expression cassette by homologous recombination. *K1L*-marker, *K1L* gene sequence including authentic promoter, Pᵥᵥ, vaccinia virus specific promoter, MCS, multiple cloning site. 1 and 2, potential undesired homologous recombination events using conventional K1L selection (indicated by dotted lines). (B) Removal of K1L sequences from the MVA genome. On top, *Hind*III restriction map of MVA genome, below, representation of ORFs at site of deletion II within MVA genome, including fragmented *K1L* gene (hatched boxes). pII_{new}LZ-gpt-del: *K1L* deletion plasmid, carrying MVA sequences flanking the 261 bp K1L fragment (N2L and *K2L* gene sequences), N2L-rep, 315 bp repeat of 5' region of N2L ORF, P11, vaccinia virus late promoter, P7.5, vaccinia virus early/late promoter, LacZ, *E. coli* LacZ gene encoding for β-galactosidase, gpt, *E*. *coli* gpt gene encoding xanthin-guanine-phosphoribosyl-transferase. On bottom, representation of deletion II locus in MVA-II_{new}. (C) New MVA transfer plasmid pIIIΔHR. Flank-I and flank-II refer to MVA-DNA sequences which are essential to target insertion of foreign genes to the site of deletion III within the MVA genome. *K1L*-marker, *K1L* gene sequence including authentic promoter, Pᵥᵥ, vaccinia virus specific promoter, MCS, multiple cloning site. Two copies of identical *lacZ*-derived DNA fragments (del) are located on either side of the *K1L* marker gene for subsequent removal of the selectable marker from recombinant MVA by homologous recombination.

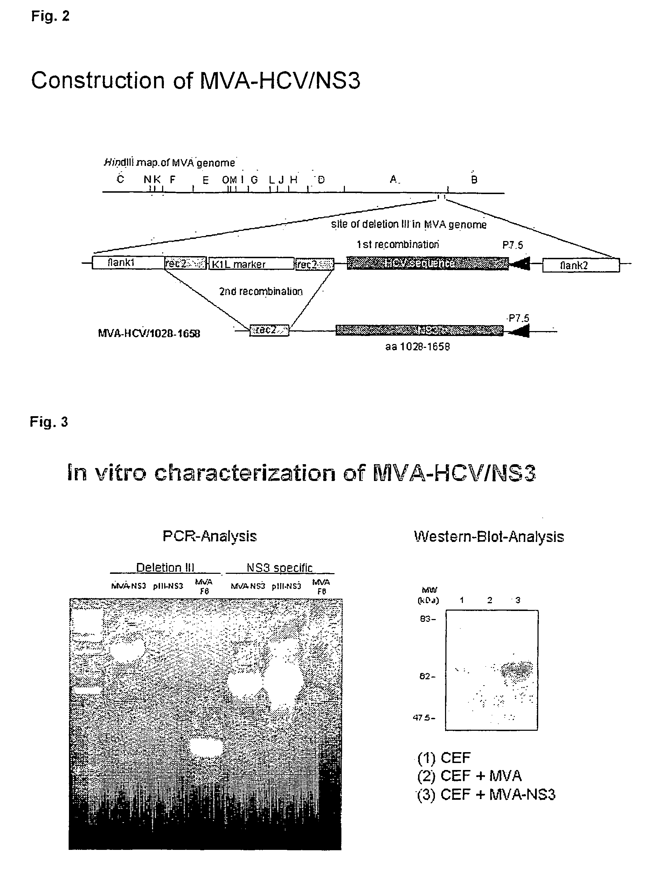

**Figure 2****: Construction and genomic structure of recombinant MVA containing gene sequences for HCV nonstructural protein** 3. A schematic map of the MVA genome for the restriction endonuclease *Hind*III is shown on top. HCV coding sequence was placed under the transcriptional control of vaccinia virus early/late promoter P7.5 and inserted by homologous recombination at the site of deletion III within the MVA genome. Flank 1 and flank 2 refer to MVA-DNA sequences that are adjacent to the site of deletion III and serve to target the recombination into the MVA genome. Rec2 indicates the positions of 283-bp repetitive MVA-DNA sequences corresponding to the right end of flank 1 and allowing removal of the K1L selectable marker from the genome of final recombinant viruses by homologous recombination. Representation of the genomic structure of recombinant MVA-HCV-NS3 is shown at the bottom.
**Figure 3****: In vitro characterization of MVA-P7.5-NS3**

Left panel: PCR analysis of viral DNA monitoring for gene sequences inserted at the site of deletion III and NS3 specific sequences. Genomic DNA of wild-type MVA, MVA-HCV-NS3 and transfer plasmid pIII-dHR-P7.5-NS3 served as template DNA for amplification of distinctive DNA fragments which were separated by agarose gel electrophoresis. The 1-kB-Ladder (Gibco) was used as marker (lane 1). Right panel: CEF cells were infected with 10 IU/cell of MVA or MVA-P7.5-NS3 and harvested at 24 h post infection. Proteins from cell lysates were separated by SDS-10% PAGE and analysed by Western blot using a polyclonal HCV specific antiserum. Position and molecular masses (in kDa) of protein standard is shown in lane MW.

The following examples are intended to contribute to a better understanding of the present invention. However, it is not intended to give the impression that the invention is confined to the subject-matter of the example.

### EXAMPLES

To improve the transient *K1L* selection technique the inventors adopted two measures: construction of MVA without *K1L* (as an example: MVA (II_{new}), Fig. 1B) and design of a new DNA vector construct (transfer plasmid) (example: pIIIΔHR, Fig. 1C).

### 1. Growing and purification of the viruses

### 1.1 Growing of the MVA and the MVA mutant virus

The MVA virus is a greatly attenuated vaccinia virus produced by serial passages of the original CVA strain on chicken embryo fibroblast (CEF) cultures. For a general review of the history of the production, the properties and the use of the MVA strain of vaccinia, reference may be made to the summary published by Mayr et al. in Infection 3, 6-14 [1975]. Owing to the adaptation to CEF, growth of the MVA virus on other cell systems is greatly restricted. Exceptionally, baby hamster kidney cells (BHK-21), a well characterized, easily maintained cell line, supports MVA growth and as proficient expression of recombinant genes as the highly efficient CEF and has been recommended for standardized MVA propagation during the development of expression vectors and live recombinant vaccines (Drexler et al. 1998, J. Gen. Virol., 79, 347-352).

The MVA virus was normally grown on CEF cells, the host cell for which it had been adapted. To prepare the CEF cells, 11-days old embryos were isolated from incubated chicken eggs, the extremities were removed, and the embryos were cut into small pieces and slowly dissociated in a solution composed of 25% trypsin at room temperature for 2 hours. The resulting cell suspension was diluted with one volume of medium I (MEM Eagle, for example obtainable from Gibco, Basle, Switzerland; Order No. 072-1500) containing 5% fetal calf serum (FCS), penicillin (100 units/ml), streptomycin (100 mg/ml) and 2 mM glutamine and filtered through a cell screen (for example obtainable from Technomara AG, Zurich, Switzerland, Order No. Bellco 1985, 150 mesh), and the cells were sedimented by centrifugation at 2000 rpm in a bench centrifuge (Hermle KG, D-7209 Gosheim, FRG) at room temperature for 5 minutes. The cell sediment was taken up in 1/4 of the original volume of medium I, and the CEF cells obtained in this way were spread on cell culture dishes. They were left to grow in medium I in a CO₂ incubator at 37°C. for 1-2 days, depending on the desired cell density, and were used for infection either directly or after 1-2 further cell passages. A clear description of the preparation of primary cultures can be found in the book by R.I. Freshney, "Culture of animal cell", Alan R. Liss Verlag, New York [1983] Chapter 11, page 99 et seq.

The MVA mutant of the present invention is routinely propagated in CEF cells or in baby hamster kidney BHK-21 (American Type Culture Collection ATCC CCL-10) cells which were grown in minimal essential medium (MEM) supplemented with 10% fetal calf serum (FCS). BHK-21 cells were maintained in a humidified air-5% CO₂ atmosphere at 37 °C.

The viruses were used for infection as follows. Cells were cultured in 175 cm² cell culture bottles. At 80-90% confluence, the medium was removed and the cells were incubated for one hour with an MVA virus suspension (0.01 infectious particles (=pfu) per cell, 0.01 ml/cm²) in phosphate-buffered saline (PBS/Dulbecco, for example Animed AG, Muttenz, Switzerland, Order No. 23.100.10). Then medium was added (0.2 ml/cm²) and the bottles were incubated at 37°C for 2-3 days until about 80% of the cells had rounded. The virus lysates were stored with the cells and medium, without treatment, in the cell culture bottles at -30°C before further processing (purification etc.)

### 1.2 Purification of the viruses

The purification steps undertaken to obtain a virus preparation which was as pure as possible and free from components specific to the host cell were identical for the MVA and WR viruses (Joklik, Virology 18, 9-18 [1962], Zwartouw et al., J. gen. Microbiol. 29, 523-529 [1962]). The cell cultures which had been infected and then stored at -30°C. were thawed, the residual cells were shaken off or scraped off the plastic substrate, and cells and virus were removed from the medium by centrifugation (Sorvall centrigue, GSA rotor, 1 hour at 5000 rpm and 10° C.). The sediment, composed of viral and cell particles, was suspended once in PBS (10-20 times the volume of the sediment), and the suspension was centrifuged as above. The new sediment was suspended in 10 times the volume of RSB buffer (10mM Tris-HCl pH 8.0, 10mM KCl, 1mM MgCl2), and the suspension was briefly treated with ultrasound (Labsonic 1510 equipped with a 4 mm diameter tip, obtainable from Bender and Hobein, Zürich, Switzerland; 2x10 seconds at 60 watts and room temperature) in order to disintegrate remaining still intact cells and to liberate the virus particles from the cell membranes. The cell nuclei and the larger cell debris were removed in the subsequent brief centrifugation of the suspension ( Sorvall GSA rotor obtainable from DuPont Co., D-6353 Bad Nauheim, FRG; 3 minutes at 3000 rpm and 10° C.). The sediment was once again suspended in RSB buffer, treated with ultrasound and centrifuged, as described above. The collected supernatants containing the free virus particles were combined and layered over a pad composed of 10 ml of 35% sucrose in 10mM Tris-HCl, pH 8.0, and centrifuged in a Kontron TST 28.38/17 rotor (Kontron Instrumente, Zurich, Switzerland; corresponds to a Beckman SW 27 rotor) for 90 minutes with 14,000 rpm at 10° C.). The supernatant was decanted, and the sediment containing the virus particles was taken up in 10ml of 10mM Tris-HCl, pH8.0, homogenized by brief treatment with ultrasound(2x10 seconds at room temperature, apparatus as described above), and applied to a stepped gradient for further purification. The steps of the gradient were each composed of 5 ml of sucrose in 10mM Tris-HCl, pH 8.0 (sucrose concentration steps: 20%, 25%, 30%, 35% and 40%). The gradient was centrifuged in a Kontron TST 28.38/17 rotor at 14,000 rpm 10° C. for 35 minutes. After this centrifugation, several discrete zones containing virus particles were visible in the region of the gradient between 30% and 40% sucrose. This region was siphoned off from the gradient (10 ml), the sucrose solution was diluted with PBS (20 ml) and the virus particles were sedimented therefrom by centrifugation (Kontron TST 28.38/17 rotor, 90 minutes at 14,000 rpm, 10° C). The sediment, which now consisted mostly of pure virus particles, was taken up in PBS in such a way that the virus concentrations corresponded on average to 1-5 x 10⁹ pfu/ml. The purified virus stock solution was used either directly or diluted with PBS for the subsequent experiments.

### 2. Construction and characterization of mutant MVA viruses

### 2.1. Construction of K1L deletion plasmid

In order to delete the remaining 263bp *K1L* sequence from the MVA genome together with promoter sequences of MVA ORF 022L (total deleted sequences comprise nt 20719 to 21169 of the MVA genome as described in Antoine, G., F. Scheiflinger, F. Dorner, and F. G. Falkner. 1998. Virology 244:365-396), the inventors constructed the deletion plasmid pII_{new}LZ-gpt-del: Flank 1 of pUCII-LZ (14), a plasmid previously used for homologous recombination into the site of deletion II within the MVA genome, was substituted by a new flank (K2L), that did not contain *K1L* gene sequences and had been isolated by PCR from MVA genomic DNA using primers IIflnewA: 5' CAG CTG CAG CGG CCG CCT TAC ACC GTA CCC 3' (SEQ ID NO: 1) and IIfinewB: 5' CAG GCA TGC GTA GAA CGT AGA TCC GG 3' (SEQ ID NO: 2). Additionally, a 315 bp repeat of the 5' region of the N2L open reading frame (ORF) (also isolated by PCR, using primers delIIA: 5' CAG CTG CAG CCA TAA TGG TCA ATC GCC 3' (SEQ ID NO: 3) and delIIB: 5' CAG GCG GCC GCG GTA TTC GAT GAT TAT TTT TAA CAA AAT AAC 3', (SEQ ID NO: 4)) was inserted downstream of the LacZ-gpt-cassette, yielding pII_{new}LZ-gpt-del and allowing for deletion of the marker cassette upon homologous recombination of N2L gene sequences.

### 2.2 Generation of MVA mutants

MVA (II_{new}) was generated by transfection of pII_{new}LZ-gpt-del DNA into primary chicken embryo fibroblasts (CEF) infected with MVA (clonal isolate F6), followed by selection of β-galactosidase producing viruses in the presence of mycophenolic acid as described previously (7). After selection of recombinant MVA, selective pressure was removed and marker free viruses isolated (Fig. 1B).

### 3. Generation of recombinant MVA viruses

### 3.1. Construction of vector plasmids

Additionally, to eliminate the possibility of homologous recombination between MVA genomic sequences and MVA repeat sequences in MVA transfer plasmids we designed a new plasmid pIIIΔHR that contains the *K1L* marker cassette now flanked by two short 216 bp repetitive sequences derived from the *lacZ* gene.

The *K1L* marker cassette, the complete *K1L* coding sequence under transcriptional control of its authentic promoter, was amplified by PCR as 1100-bp DNA fragment from genomic DNA of vaccinia virus strain Western Reserve (kindly provided by Dr. B. Moss, LVD-NIH, Bethesda MD, USA) using the oligonucleotides KIL-5'-3 CAG CAG CCC GGG TGC GAT AGC CAT GTA TCT ACT AAT CAG (SEQ ID NO: 5) and K1L-3'-1 CAG CAG CCC GGG GGA AAT CTA TCT TAT ATA CAC (SEQ ID NO: 6) (sites for the restriction enzyme *Sma*I are underlined).

This *K1L* marker cassette with the direct repeats on either side was excised from pΔK1L that has been described previously (12) and was inserted into pIIIdHR, replacing the *K1L* marker and flank I repeat to generate pIIIΔHR (Fig 1C). The genome of final MVA recombinant viruses will now contain the target gene sequences and one *LacZ* gene fragment. Insertion of additional foreign DNA might appear to result in less "clean" vector viruses, yet this inert sequence may serve as a general genetic marker for convenient identification of recombinant MVA.

### 3.2. Formation and isolation of recombinant MVA

Finally, the impact of these alterations on recombination efficiencies in infection/transfection experiments was determined. CEF cells were infected with MVA (F6) or MVA (II_{new}) and transfected with pIIIdHR-gfp or pIIIΔHR-gfp. Samples were harvested after 48 hours, and aliquots were used to infect RK13 monolayers. After three days infected monolayers were harvested completely and material subjected to a second passage on RK13 cells. Three days post infection numbers of visible/gfp fluorescent cell aggregates were determined by light/UV light microscopy. The inventors then used this data to calculate the percentage of correct recombination events, i.e. insertion of the *K1L* marker cassette and the *gfp* gene into MVA (Table 1). Using the conventional selection system (MVA (F6) and pIIIdHR-gfp) generated by the inventors, they found 30.5% of all foci recombinant for *K1L* and gfp after the second RK13 passage. Deletion of the remaining K1L sequence from the MVA genome slightly improved correct recombination events (45%, MVA (II_{new}) and pindHR-gfp). Whereas removal of flank 1 repeat sequence from the MVA transfer plasmid resulted in a clear increase of desired recombination events (71%) even using MVA still harboring left-over *K1L* sequences. The inventors obtained virtually 100% efficiency when using new MVA and gfp-transfer plasmid (MVA (II_{new}) and pIIIΔHR-gfp). One first "blind" passage on RK13 cells was performed before plating dilutions and determining the number of foci, because it was observed, that when counting GFP positive cell aggregations in the first RK13 passage the outcome suggested higher numbers of correct recombination events, yet about half of these virus isolates when brought into the next passage was unable to induce GFP-positive virus foci. This observation is probably based on unstable single recombination events and/or transient *gfp* expression from carry-over plasmid DNA.

Taken together the inventors are able to further substantially improve the *K1L*-based selection technique by (i) removal of the remaining *K1L* sequences within the MVA genome and (ii) design of new MVA transfer plasmids carrying homologous non-MVA sequences for deletion of the transient marker gene. Each measure by itself already improved generation of the desired recombinant viruses, albeit to different extents. Combined use of a MVA backbone virus free of *K1L* sequences with modified MVA vector plasmids resulted in very efficient selection and precise gene transfer into the targeted genome site, and allows for isolation of practically only MVA recombinant viruses.

### 4. Method of generating/selecting rMVA

The successful construction of a recombinant MVA (rMVA) expressing the nonstructural 3 (NS3) open reading frame (ORF) of the HCV-J strain (genotype 1b) may serve as example for the advantageous use of our new technique to generate rMVA on the basis of an MVA parent virus lacking intrinsic K1L sequences (MVA-II_{new}) and using transfer plasmids with heterologous, non-MVA repetitive sequence elements (LacZ). Originally, we had attempted to generate such a rMVA for expression of the HCV NS3 gene (MVA-HCV/NS3) of the HCV-J strain (genotype 1b) using our conventional host range selection protocol as described in Staib C., et al. 2000, Biotechniques 28:1137-1148.

A plasmid containing the HCV cDNA derived from a Japanese patient with chronic hepatitis and obtained from Dr. Kunitada Shimotohno, Kyoto University, Japan (Kato et al. 1990, PNAS 87:9524-9528) served to prepare the NS3 gene (encoding HCV polyprotein amino acids 1028-1658) using PCR amplification, and to clone the corresponding PCR product into the MVA transfer vector pIII-dHR-P7.5 (Fig. 2). After infection of CEF monolayers with MVA (clonal isolate F6) and transfection with pIII-dHR-P7.5-NS3, followed by several plaque purification steps on RK13 cells, using K1L as selectable marker, we were unable to obtain bona fide recombinant viruses. We isolated either rMVA harboring only the K1L sequence stably integrated within the site of deletion 3 (del 3) of the MVA genome, or were unable to obtain stably growing virus progeny at all. As a remedy, we then decided to use our improved new K1L selection technique, recloned the NS3 gene sequence into the new MVA vector plasmid pIII-ΔHR-P7.5, and used the improved K1L-free isolate MVA-II_{new} as receptor virus for integration of the expression cassette by homologous recombination. Indeed, this change of strategy allowed to generate and isolate the desired recombinant virus MVA-HCV/NS3 within only a few plaque purification steps. Figure 3 depicts the *in vitro* characterization of this rMVA by PCR analysis (left panel), monitoring for the correct insertion of the NS3 ORF precisely at the site of del 3 of the MVA genome and complete removal of the K1L selectable marker cassette. Further more, the newly generated recombinant MVA-HCV/NS3 is able to produce the correct NS3 polypeptides upon infection of CEF cells, as demonstrated by Western blot analysis (Fig. 3, right panel). MVA-HCV/NS3 is currently being evaluated as a promising candidate vector virus for the development of prophylactic and/or therapeutic vaccines against HCV infection of humans. Thus, our improved methodology, described in this patent application, already allowed us to obtain a new important rMVA vector construct which if to be generated by standard procedures would have been more cumbersome if not impossible.

### References

**1.** Amara, R. R., Villinger, F., Altman, J. D., Lydy, S. L., SP, O. N., Staprans, S. I., Montefiori, D. C., XU, Y., Herndon, J. G., Wyatt, L. S., Candido, M. A., Kozyr, N. L., Earl, P. L., Smith, J. M., Ma, H. L., Grimm, B. D., Hulsey, M. L., Miller, J., McClure, H. M., McNicholl, J. M., Moss, B., and Robinson, H. L. (2001). Control of a Mucosal Challenge and Prevention of AIDS by a Multiprotein DNA/MVA Vaccine. Science 292(5514), 69-74.
**2.** Belyakov, I.M., L.S. Wyatt, J.D. Ahlers, P. Earl, C.D. Pendleton, B.L. KelsaII, W. Strober, B. Moss and J.A. Berzofsky. 1998. Induction of a mucosal cytotoxic T-lymphocyte response by intrarectal immunization with a replication-deficient recombinant vaccinia virus expressing human immunodeficiency virus 89.6 envelope protein. J. Virol. 72:8264-8272.
**3.** Drexler, I., E. Antunes, M. Schmitz, T. Wolfel, C. Huber, V. Erfle, P. Rieber, M. Theobald and G. Sutter. 1999. Modified vaccinia virus Ankara for delivery of human tyrosinase as melanoma-associated antigen: induction of tyrosinase- and melanoma-specific human leukocyte antigen A*0201-restricted cytotoxic T cells in vitro and in vivo. Cancer Res. 59:4955-4963.
**4.** Hirsch, V.M., Fuerst, T.R., Sutter, G., Carroll, M.W., Yang, L.C., Goldstein, S., Piatak, M., Elkins, W.R., Alvord, W.G., Montefiori, D.C., Moss, B. and Lifson, J.D. (1996) Patterns of viral replication correlate with outcome in simian immunodeficiency virus (SIV)- infected macaques: Effect of prior immunization with a trivalent SIV vaccine in modified vaccinia virus Ankara. J. Virol. 70, 3741-3752.
5. Moss, B. 1996. Genetically engineered poxviruses for recombinant gene expression, vaccination, and safety. Proc. Natl. Acad. Sci. U.S.A. 93:11341-11348.
**6.** Schneider, J., S.C. Gilbert, T.J. Blanchard, T. Hanke, K.J. Robson, C.M. Hannan, M. Becker, R. Sinden, G.L. Smith and A.V. Hill. 1998. Enhanced immunogenicity for CD8+ T cell induction and complete protective efficacy of malaria DNA vaccination by boosting with modified vaccinia virus Ankara. Nat Med. 4: 397-402.
7. Sutter, G. and B. Moss. 1992. Nonreplicating vaccinia vector efficiently expresses recombinant genes. Proc. Natl. Acad. Sci. USA 89:10847-10851.
8. Sutter, G., L. S. Wyatt, P. L. Foley, J. R. Bennink and B. Moss. 1994. A recombinant vector derived from the host range-restricted and highly attenuated MVA strain of vaccinia virus stimulates protective immunity in mice to influenza virus. Vaccine 12:1032-1040.
9. Corona Gutierrez CM, Tinoco A, Lopez Contreras M, Navarro T, Calzado P, Vargas L, Reyes L, Posternak R, Rosales R. (2002). Clinical protocol. A phase II study: efficacy of the gene therapy of the MVA E2 recombinant virus in the treatment of precancerous lesions (NIC I and NIC II) associated with infection of oncogenic human papillomavirus. Hum Gene Ther 13(9):1127-40
**10.** Hanke T, McMichael AJ, Mwau M, Wee EG, Ceberej I, Patel S, Sutton J, Tomlinson M, Samuel RV. (2002). Development of a DNA-MVA/HIVA vaccine for Kenya. Vaccine 20(15):1995-8
11. Hill AV, Reece W, Gothard P, Moorthy V, Roberts M, Flanagan K, Plebanski M, Hannan C, Hu JT, Anderson R, Degano P, Schneider J, Prieur E, Sheu E, Gilbert SC. (2000).DNA-based vaccines for malaria: a heterologous prime-boost immunisation strategy. Dev Biol (Basel) 104:171-9
12. Staib, C., Drexler, I., Qhlmann, M., Wintersperger, S., Erfle, V., Sutter, G. (2000) Transient host range selection for genetic engineering of modified vaccinia virus Ankara. BioTechniques 6,1137-42, 1144-6, 1148.
**13.** Tscharke, D. C., and Smith, G. L. (2002). Notes on Transient Host Range Selection for Engeneering Vaccinia Virus Strain MVA. BioTechniques 33: 186-188.
14. Sutter, G., Ohlmann, M., and Erfle, V. 1995. Non-replicating vaccinia vector efficiently expresses bacteriophage T7 RNA polymerase. FEBS Lett. 371(1):9-13.

**Table 1: Recombination efficiency using conventional and new MVA and transfer plasmids.**

| | MVA (F6) | MVA (II_{new}) |
|---|---|---|
| pIIIdHR-gfp | 30.5 % (27*/88** - 6/20) | 45% (48/121 - 37/73) |
| pIIIΔHR-gfp | 71% (78/108 -16/23) | 99.7% (154/155 - 25/25) |

Percentages were calculated as ratio of gfp-expressing foci* to foci visible under light microscope**. Numbers in parentheses show absolute number of foci counted in two different 10-fold dilutions of each sample and are representative for three independent experiments.

## Claims

1. A DNA-vector construct, which comprises sequences encoding the Vaccinia Virus (VV) *K1L* gene or a functionally equivalent gene, wherein the VV *K1L* and foreign protein coding regions are flanked by DNA-sequences, flanking a non-essential site within the MVA genome, wherein the non-essential site is the site of the engineered *K1L* deletion within the MVA genome.

2. The DNA-vector construct of claim 1, wherein the vector comprises the following functionally linked components:
- the *K1L* marker gene comprising *K1L* coding sequence of VV and a transcription unit, preferably comprising the transcriptional control sequences of its authentic promoter,
- two DNA sequences flanking the *K1L* marker gene for subsequent removal of the marker from recombinant MVA by homologous recombination, preferably two identical inert (e.g. E. coli *lacZ* derived) DNA fragments,
- a cloning site, preferably multiple cloning site, in which a heterologous gene has been optionally inserted,
- a promoter for vaccinia virus specific transcription, preferably a vaccinia virus derived promoter, or a heterologous poxvirus promoter which allows for vaccinia virus specific transcription, or a synthetic promoter which allows for vaccinia virus specific transcription,
which components are flanked by two MVA-DNA sequences, which are essential to targeted insertion of foreign genes to the site of the engineered *K1L* deletion within the MVA genome.

3. A MVA mutant, wherein the *K1L* gene sequences and its promoter sequences in the MVA genome have been inactivated to avoid homologous recombination with the DNA-vector construct of claim 1 by deletion or mutation.

4. A host cell, which has been infected with the MVA of claim 3.

5. The host cell of claim 4, which is a eucaryotic cell.

6. The eucaryotic cell of claim 5, which is a chicken fibroblast cell, a quail fibroblast cell, a QT-9 cell, a BHK-21 cell, a BS-C-1 cell, a MA104 cell, a CV-1 cell, a Vero cell, a MRC-5 cell, a B-cell or a human primary cell (e.g. primary fibroblast cells, dendritic cells).

7. A method of generating recombinant MVA, comprising the steps of:
- Infecting host cells of MVA with the MVA mutant of claim 3,
- transfecting the host cells with a DNA-vector construct, which comprises sequences, encoding the Vaccinia Virus (VV) *K1L* gene or a functionally equivalent gene; and
- selecting restored MVA by growth on rabbit kidney RK-13 cells, or any other cell type that essentially requires *K1L* gene function to allow for productive growth of mutant MVA of claim 3.

8. The method of claim 7, wherein the construct further comprises DNA sequences coding for a foreign protein or a functional part therof.

9. The method of claim 8, wherein the foreign protein is a heterologous protein derived from the group consisting of therapeutic polypeptides and polypeptides of pathogenic agents and functional parts thereof.

10. The method of claim 9, wherein the therapeutic polypeptide is derived from the group consisting of secreted proteins, e.g. polypeptides of antibodies, chemokines, cytokines or interferons.

11. The method of claim 9, wherein the pathogenic agent is derived from the group consisting of viruses, bacteria, protozoa and parasites as well as tumor cells or tumor cell associated antigens and functional parts thereof.

12. The method of claim 11, wherein the viruses are selected from the group consisting of influenza viruses, measles and respiratory syncytial viruses, dengue viruses, human immunodeficiency viruses, human hepatitis viruses, herpes viruses, or papilloma viruses.

13. The method of claim 11, wherein the protozoa is Plasmodium falciparum.

14. The method of claim 11, wherein the bacteria is tuberculosis-causing Mycobacteria.

15. The method of claim 11, wherein the tumor cell associated antigen is selected from the group consisting of melanoma-associated differentiation antigens, e.g. tyrosinase, tyrosinase-related proteins 1 and 2, of cancer testes antigens, e.g. MAGE-1,-2,-3, and BAGE, and of non-mutated shared antigens overexpressed on tumors, e.g. Her-2/neu, MUC-1, and p53.

16. The method of any one of claims 7-15, wherein the VV *K1L* and foreign protein coding regions are each flanked by DNA-sequences, flanking a non-essential site within the MVA genome.

17. The method of claim 16, wherein the non-essential site is the site of deletion III in the MVA genome, the site of the engineered *K1L* deletion within the MVA genome or any non-essential site within the genome of mutant MVA according to claim 3.

18. The method of any one of claims 7-17, wherein the vector comprises the following functionally linked components:
- the *K1L* marker gene comprising *K1L* coding sequence of VV and a transcription unit, preferably comprising the transcriptional control sequences of its authentic promoter,
- two DNA sequences flanking the *K1L* marker gene for subsequent removal of the marker from recombinant MVA by homologous recombination, preferably two identical inert (e.g. E. coli *lacZ* derived) DNA fragments,
- a cloning site, preferably multiple cloning site, in which a heterologous gene has been optionally inserted,
- a promoter for vaccinia virus specific transcription, preferably a vaccinia virus derived promoter, or a heterologous poxvirus promoter which allows for vaccinia virus specific transcription, or a synthetic promoter which allows for vaccinia virus specific transcription,
which components are flanked by two MVA-DNA sequences, which are essential to targeted insertion of foreign genes to any non-essential site within the genome of mutant MVA according to claim 3, to the site of deletion III within the MVA genome, or to the site of the engineered *K1L* deletion within the MVA genome.

19. The method of any of claims 7-18, wherein the DNA vector construct is a a plasmid.

## Patentansprüche

1. DNA-Vektorkonstrukt, welches die für das Vaccinia Virus (VV) *K1L*-Gen oder ein funktionell equivalentes Gen kodierende Sequenzen umfasst, wobei die für das VV *K1L* und das Fremdprotein kodierende Abschnitte durch DNA-Sequenzen flankiert werden, die eine nicht-essentielle Stelle im MVA-Genom flankieren, wobei die nicht-essentielle Stelle die Stelle der veränderten *K1L*-Deletion im MVA-Genom ist.

2. DNA-Vektorkonstrukt nach Anspruch 1, wobei der Vektor die nachfolgenden, funktionell verbundenen Bestandteile umfasst:
- das *K1L*-Markergen, welches die für *K1L* kodierende Sequenz von VV und eine Transkriptionseinheit umfasst, die bevorzugt die Transkriptionskontrollsequenzen des ursprünglichen Promotors aufweist,
- zwei DNA-Sequenzen, die das *K1L*-Markergen zum nachfolgenden Entfernen des Markers aus dem rekombinanten MVA durch homologe Rekombination flankieren, bevorzugt zwei identische inerte DNA-Fragmente (z.B. von E. coli *lacZ* abstammend),
- eine Klonierungsstelle; bevorzugt eine multiple Klonierungsstelle, in welche ein heterologes Gen wahlweise insertiert wurde;
- einen Promotor zur Vaccinia Virus spezifischen Transkription, bevorzugt einen von einem Vaccinia Virus stammenden Promotor, oder einen heterologen Poxviruspromotor, der eine Vaccinia Virus spezifische Transkription ermöglicht, oder einen synthetischen Promotor, der eine Vaccinia Virus spezifische Transkription ermöglicht,
wobei die Bestandteile von zwei MVA-DNA-Sequenzen flankiert sind, die für eine Target spezifische Insertion von Fremdgenen an die Stelle der veränderten *K1L-*Deletion im MVA-Genom essentiell sind.

3. MVA-Mutante, wobei die *K1L*-Gensequenzen und seine Promotorsequenzen im MVA-Genom durch Deletion oder Mutation inaktiviert wurden, um eine homologe Rekombination mit dem DNA-Vektorkonstrukt von Anspruch 1 zu verhindern.

4. Wirtszelle, die mit dem MVA nach Anspruch 3 infiziert ist.

5. Wirtszelle nach Anspruch 4, die eine Eukaryontenzelle ist.

6. Eukaryontenzelle nach Anspruch 5, die eine Hühnerfibroblastenzelle, eine Wachtelfibroblastenzelle, eine QT-9-Zelle, eine BHK-21-Zelle, eine BS-C-1-Zelle, eine MA104 Zelle, eine CV-1-Zelle, eine Verozelle, eine MRC-5-Zelle, eine B-Zelle oder eine primäre Humanzelle (z.B. primäre Fibroblastenzellen, dendritische Zellen) ist.

7. Verfahren zum Erzeugen von rekombinantem MVA, umfassend die Schritte:
- Infizieren von Wirtszellen von MVA mit der MVA-Mutante nach Anspruch 3,
- Transfizieren der Wirtszellen mit einem DNA-Vektorkonstrukt, das Sequenzen umfasst, die für das Vaccinia Virus (VV) *K1*L-Gen oder einem funktionell äquivalenten Gen kodieren; und
- Auswählen des wieder hergestellten MVA durch Wachstum auf RK-13-Kaninchennierenzellen, oder irgendeinem anderen Zelltyp, der notwendigerweise die *K1L*-Genfunktion benötigt, um ein produktives Wachstum der MVA-Mutante nach Anspruch 3 zu ermöglichen.

8. Verfahren nach Anspruch 7, wobei das Konstrukt weiterhin DNA-Sequenzen umfasst, die für ein Fremdprotein oder einem funktionellen Teil hiervon kodieren.

9. Verfahren nach Anspruch 8, wobei das Fremdprotein ein heterologes Protein ist, welches von der Gruppe abgeleitet ist, bestehend aus therapeutischen Polypeptiden und Polypeptiden von pathogenen Mitteln und funktionellen Teilen hiervon.

10. Verfahren nach Anspruch 9, wobei das therapeutische Polypeptid abgeleitet ist aus der Gruppe, bestehend aus siezernierten Proteinen, beispielsweise Polypeptiden von Antikörpern, Chemokinen, Zytokinen oder Interferonen.

11. Verfahren nach Anspruch 9, wobei das pathogene Mittel abgeleitet ist aus der Gruppe, bestehend aus Viren, Bakterien, Protozoen und Parasiten als auch aus Tumoren oder Tumorzell-assoziierten Antigenen und funktionellen Teilen hiervon.

12. Verfahren nach Anspruch 11, wobei die Viren ausgewählt werden aus der Gruppe, bestehend aus Influenzaviren, Masern- und respiratorischen synzytialen Viren, Dengueviren, Human-Immunodeffizienzviren, Human-Hepatitisviren, Herpesviren oder Papillomaviren.

13. Verfahren nach Anspruch 11, wobei die Protozoen Plasmodium falciparum sind.

14. Verfahren nach Anspruch 11, wobei die Bakterien Tuberkulose verursachende Mykobakterien sind.

15. Verfahren nach Anspruch 11, wobei das Tumorzell-assoziierte Antigen ausgewählt wird aus der Gruppe, bestehend aus Melanom-assozierten Differenzierungsantigenen, beispielsweise Tyrosinase, Tyrosinase verwandten Proteinen 1 und 2, aus Hodenkrebsantigenen, z.B. MAGE-1, -2, und -3, und BAGE und nicht mutierten Shared-Antigenen, die auf Tumoren überexprimiert werden, z.B. Her-2/neu, MUC-1 und p53.

16. Verfahren nach irgendeinem der Ansprüche 7 bis 15, wobei die für das VV *K1L-*und das Fremdprotein kodierenden Abschnitte jeweils von DNA-Sequenzen flankiert werden, die eine nicht-essentielle Stelle im MVA-Genom flankieren.

17. Verfahren nach Anspruch 16, wobei die nicht-essentielle Stelle die Stelle der Deletion III im MVA-Genom, die Stelle der veränderten *K1L*-Deletion im MVA-Genom oder irgend eine andere nicht essentielle Stelle im Genom des mutierten MVA nach Anspruch 3 ist.

18. Verfahren nach irgendeinem der Ansprüche 7 bis 17, wobei der Vektor die nachfolgenden funktionell verbundenen Bestandteile umfasst:
- das *K1L*-Markergen, welches die für *K1L* kodierende Sequenz von VV und eine Transkriptionseinheit umfasst, die bevorzugt die Transkriptionskontrollsequenzen des ursprünglichen Promotors aufweist,
- zwei DNA-Sequenzen, die das *K1L*-Markergen zum nachfolgenden Entfernen des Markers aus dem rekombinanten MVA durch homologe Rekombination flankieren, bevorzugt zwei identische inerte DNA-Fragmente (z.B. von E. coli *lacZ* abstammend),
- eine Klonierungsstelle, bevorzugt eine multiple Klonierungsstelle, in welche ein heterologes Gen wahlweise insertiert wurde;
- einen Promotor zur Vaccinia Virus spezifischen Transkription, bevorzugt einen von einem Vaccinia Virus stammenden Promotor, oder einen heterologen Poxviruspromotor, der eine Vaccinia Virus spezifische Transkription ermöglicht, oder einen synthetischen Promotor, der eine Vaccinia Virus spezifische Transkription ermöglicht,
wobei die Bestandteile von zwei MVA-DNA-Sequenzen flankiert sind, die für eine Target spezifische Insertion von Fremdgenen an irgendeine nicht-essentielle Stelle im Genom des mutierten MVA nach Anspruch 3, an die Stelle der Deletion III im MVA-Genom, oder an die Stelle der veränderten *K1L*-Deletion im MVA-Genom essentiell sind.

19. Verfahren nach irgendeinem der Ansprüche 7 bis 18, wobei das DNA-Vektorkonstrukt ein Plasmid ist.

## Revendications

1. Construction génique d'ADN-vecteur, qui comprend des séquences codant le gène *K1L* du virus de la vaccine (VV) ou un gène fonctionnellement équivalent, dans laquelle les régions codantes du *K1L* du VV et de protéines étrangères sont flanquées de séquences ADN, flanquant un site non essentiel dans le génome du MVA, dans laquelle le site non essentiel est le site de la délétion du *K1L* modifié dans le génome du MVA.

2. Construction génique d'ADN-vecteur selon la revendication 1, dans laquelle le vecteur comprend les composants fonctionnellement liés suivants :
- le gène marqueur *KIL* comprenant la séquence codante du *KIL* du VV et une unité de transcription, de préférence comprenant les séquences de contrôle transcriptionnel de son promoteur natif,
- deux séquences ADN flanquant le gène marqueur *KIL* pour l'élimination ultérieure du marqueur du MVA recombinant par recombinaison homologue, de préférence deux fragments d'ADN (par exemple dérivé de E. coli *lacZ*) identiques et inertes,
- un site de clonage, de préférence un site de clonage multiple, dans lequel un gène hétérologue a été facultativement inséré,
- un promoteur pour la transcription spécifique du virus de la vaccine, de préférence un promoteur dérivé du virus de la vaccine, ou un promoteur hétérologue de poxvirus qui permet la transcription spécifique du virus de la vaccine, ou un promoteur synthétique qui permet la transcription spécifique du virus de la vaccine,
lesquels composants sont flanqués de deux séquences de MVA-ADN, qui sont essentielles à l'insertion ciblée de gènes étrangers sur le site de la délétion du *KIL* modifié dans le génome du MVA.

3. Mutant de type MVA, dans lequel les séquences du gène *KIL* et ses séquences de promoteur dans le génome du MVA ont été inactivées pour éviter une recombinaison homologue avec la construction génique d'ADN-vecteur selon la revendication 1 par délétion ou mutation.

4. Cellule hôte, laquelle a été infectée avec le MVA selon la revendication 3.

5. Cellule hôte selon la revendication 4, laquelle est une cellule eucaryote.

6. Cellule eucaryote selon la revendication 5, laquelle est une cellule fibroblastique de poulet, une cellule fibroblastique de caille, une cellule QT-9, une cellule BHK-21, une cellule BS-C-1, une cellule MA104, une cellule CV-1, une cellule Véro, une cellule MRC-5, une cellule B ou une cellule humaine primaire (par exemple des cellules fibroblastiques primaires, des cellules dendritiques).

7. Procédé de génération d'un MVA recombinant comprenant les étapes de:
- infecter des cellules hôtes de MVA avec le mutant de type MVA selon la revendication 3,
- transfecter les cellules hôtes avec une construction génique d'ADN-vecteur, qui comprend des séquences codant le gène *KIL* du virus de la vaccine (VV) ou un gène fonctionnellement équivalent ; et
- sélectionner un MVA restauré par croissance sur des cellules RK-13 de rein de lapin, ou n'importe quel autre type de cellule qui requiert essentiellement la fonction de gène *KIL* pour permettre une croissance productive du mutant de type MVA selon la revendication 3.

8. Procédé selon la revendication 7, dans lequel la construction génique comprend en outre des séquences ADN codant une protéine étrangère ou une partie fonctionnelle de celle-ci.

9. Procédé selon la revendication 8, dans lequel la protéine étrangère est une protéine hétérologue dérivée du groupe constitué par les polypeptides thérapeutiques et les polypeptides d'agents pathogènes et des parties fonctionnelles de ceux-ci.

10. Procédé selon la revendication 9, dans lequel le polypeptide thérapeutique est dérivé du groupe constitué par les protéines secrétées, par exemple les polypeptides d'anticorps, les chémokines, les cytokines ou les interférons.

11. Procédé selon la revendication 9, dans lequel l'agent pathogène est dérivé du groupe constitué par les virus, les bactéries, les protozoaires et les parasites ainsi que les cellules tumorales ou les antigènes associées à des cellules tumorales et les parties fonctionnelles de ceux-ci.

12. Procédé selon la revendication 11, dans lequel les virus sont choisis dans le groupe constitué par les virus de la grippe, le virus de la rougeole et le virus respiratoire syncytial, les virus de la dengue, les virus de l'immunodéficience humaine, les virus de l'hépatite humaine, les virus de l'herpès, ou les papillomavirus.

13. Procédé selon la revendication 11, dans lequel les protozoaires sont le Plasmodium falciparum.

14. Procédé selon la revendication 11, dans lequel les bactéries sont les mycobactéries provoquant la tuberculose.

15. Procédé selon la revendication 11, dans lequel l'antigène associé à une cellule tumorale est choisi dans le groupe constitué par les antigènes de différentiation associé au mélanome, par exemple la tyrosinase, les tyrosinase-related protein 1 et 2, les antigènes du cancer du testicule, par exemple les MAGE-1,-2,-3, et le BAGE, et les antigènes partagés, non mutés et surexprimés dans les tumeurs, par exemple les Her-2/neu, MUC-1, et p53.

16. Procédé selon l'une quelconque des revendications 7 à 15, dans lequel les régions codantes du *KIL* du VV et de protéines étrangères sont chacune flanquées de séquences ADN, flanquant un site non essentiel dans le génome du MVA.

17. Procédé selon la revendication 16, dans lequel le site non essentiel est le site de la délétion III dans le génome du MVA, le site de la délétion du *KIL* modifié dans le génome du MVA ou n'importe quel site non essentiel dans le génome du MVA mutant selon la revendication 3.

18. Procédé selon l'une quelconque des revendications 7 à 17, dans lequel le vecteur comprend les composants fonctionnellement liés suivants :
- le gène marqueur *KIL* comprenant la séquence codante du *KIL* du VV et une unité de transcription, de préférence comprenant les séquences de contrôle transcriptionnel de son promoteur natif,
- deux séquences ADN flanquant le gène marqueur *KIL* pour l'élimination ultérieure du marqueur du MVA recombinant par recombinaison homologue, de préférence deux fragments d'ADN (par exemple dérivé de E. coli *lacZ*) identiques et inertes,
- un site de clonage, de préférence un site de clonage multiple, dans lequel un gène hétérologue a été facultativement inséré,
- un promoteur pour la transcription spécifique du virus de la vaccine, de préférence un promoteur dérivé du virus de la vaccine, ou un promoteur hétérologue de poxvirus qui permet la transcription spécifique du virus de la vaccine, ou un promoteur synthétique qui permet la transcription spécifique du virus de la vaccine,
lesquels composants sont flanqués de deux séquences de MVA-ADN, qui sont essentielles à l'insertion ciblée de gènes étrangers sur n'importe quel site non essentiel dans le génome du MVA mutant selon la revendication 3, sur le site de la délétion III du génome MVA, ou sur le site de la délétion du *KIL* modifié dans le génome du MVA.

19. Procédé selon l'une quelconque des revendications 7 à 18, dans lequel la construction génique d'ADN-vecteur est un plasmide.
